# EUROPÄISCHE PATENTSCHRIFT

(11) **EP 0 389 836 B1**
(45) Veröffentlichungstag und Bekanntmachung des Hinweises auf die Patenterteilung: **21.04.1993**
(21) Anmeldenummer: 90104333.1
(22) Anmeldetag: 07.03.1990
(51) Int. Cl.: C07C 53/08, C07C 53/12, C07C 69/16, C07C 51/48, C07C 51/573, C07C 67/58

(54) **Verfahren zur Reinigung und Rückgewinnung der bei der Carbonylierung von Methanol und/oder Methylacetat und/oder Dimethylether anfallenden verunreinigten Katalysatorlösung**
Process for the purification and recovery of contaminated solution of a catalyst from the carbonylation of methanol and/or methyl acetate and/or dimethyl ether
Procédé de purification et de récupération de la solution résiduelle du catalyseur contaminé à partir de la carbonylation du méthanol et/ou d'acétate de méthyle et/ou d'éther diméthylique

(30) Priorität: 22.03.1989 DE 3909445
(43) Veröffentlichungstag der Anmeldung: 03.10.1990
(73) Patentinhaber: HOECHST AKTIENGESELLSCHAFT, 65926 Frankfurt am Main (DE)
(72) Erfinder: Erpenbach, Heinz, Dr., D-5000 Köln (DE); Lork, Winfried, D-5042 Erftstadt (DE); Weferling, Norbert, Dr., D-5030 Hürth (DE); Prinz, Peter, D-5030 Hürth (DE)

(56) Entgegenhaltungen:
- EP-A- 0 200 023
- US-A- 4 143 066
- US-A- 4 650 615

## Beschreibung

Die Erfindung betrifft ein Verfahren zur Reinigung und Rückgewinnung der bei der Carbonylierung von Methanol und/oder Methylacetat und/oder Dimethylether anfallenden verunreinigten Katalysatorlösung, welche Carbonylkomplexe des Rhodiums, quaternäre Organophosphorverbindungen als organische Promotoren, undestillierbare organische Verunreinigungen sowie Essigsäure, Essigsäureanhydrid und Ethylidendiacetat enthält.

Zur Durchführung von Hydroformylierungs- und Carbonylierungsverfahren wird als Edelmetallkatalysator Rhodium in Form vielfältiger komplexer Verbindungen eingesetzt. Da Rhodium aufgrund seiner geringen Verfügbarkeit ein sehr teures Edelmetall ist, wird über seine Rückgewinnung bzw. die Reinigung von Rhodiumkomplexen aus mit Rückständen verunreinigten Katalysatorsystemen bzw. Destillationssümpfen der oben angeführten Reaktionen mehrfach in der Literatur berichtet.

Die EP-A-0 240 703 (= US-A-4,746,640) beansprucht ein Verfahren, das es gestattet, die bei der Carbonylierung von Methylacetat und/oder Dimethylether anfallende, verunreinigte rhodiumhaltige Katalysatorlösung so zu reinigen, daß die bei der Umsetzung gebildeten undestillierbaren organischen Rückstände durch eine Flüssig-Flüssig-Extraktion mit Dialkylethern und Alkanolen mit jeweils 1-4 C-Atomen der Lösung entzogen werden. Die die organischen Rückstände enthaltende Etherphase wird einer Nachbehandlung mit Iod und/oder Methyliodid unterzogen, vom ausgefallenen restlichen Katalysatorkomplex abgetrennt und dann weiter destillativ in wiederzuverwendenden Dialkylether und wiederzuverwendendes Alkanol sowie Essigsäure, Essigsäureanhydrid, Ethylidendiacetat und undestillierbaren Rückstand getrennt. Essigsäure, Essigsäureanhydrid und Ethylidendiacetat werden mit der gereinigten Katalysatorphase sowie dem aus der Etherphase isolierten Katalysatorkomplex vereinigt, vom restlichen Ether und Alkohol befreit und als frische Katalysatorlösung der Reaktion zugeführt. In dem ausgeschleusten Rückstand der Etherphasendestillation verbleiben nur noch < 0,1 % des in die Extraktion eingesetzten Rhodiums.

Der hohe Preis des Rhodiums gestattet nur eine verlustfreie Verwendung als Katalysator für großtechnische Verfahren. Dies gilt auch für die Reinigung und Rückgewinnung eines eingesetzten verunreinigten Rhodiumkatalysators und macht daher einen quantitativen Kreislauf unerläßlich. Dies erfordert eine Abtrennung des im Verfahren gebildeten organischen Rückstandes aus der Katalysatorlösung ohne jeden Rh-Verlust und den direkten Wiedereinsatz des gereinigten Katalysatorsystems in die Reaktion.

Das Verfahren der EP-A-0 240 703 (= US-A-4,746,640) erfüllt die aufgestellten Forderungen voll und weist mit 99,9 % eine sehr gute Rhodiumrückgewinnungsrate auf. Es erfordert jedoch eine zusätzliche Nachbehandlung der Etherphase mit Jod und nachfolgende Abtrennung der Behandlungsprodukte.

Die vorliegende Erfindung beschreibt nun ein Verfahren, das eine Reinigung der bei der Carbonylierung von Methanol und/oder Methylacetat und/oder Dimethylether eingesetzten und im Laufe des Prozesses verunreinigten Katalysatorlösung durch Extraktion mit Tri-alkylphosphan ermöglicht, wobei die Abtrennung der undestillierbaren organischen Verunreinigungen aus der Katalysatorlösung ohne Rhodiumverlust und ohne Zerstörung des Rhodium-Carbonylkomplexes sowie des Promotors erfolgt. Rh-Komplex und Promotor können ohne zusätzliche Maßnahmen dem Carbonylierungsprozeß wieder zugeführt werden. Durch den Kreislaufbetrieb des zur Aufarbeitung eingesetzten Extraktionsmittels Tri-alkylphosphan wird eine Umweltbelastung durch Abfallstoffe vermieden. Nur die im Prozeß gebildeten organischen Verunreinigungen werden ausgeschleust und können entsprechend dem Stand der Technik beseitigt werden. Der Extraktionsprozeß des Verfahrens der Erfindung kann in Form einer Flüssig-Flüssig-Extraktion durchgeführt werden. Eine kontinuierliche Arbeitsweise ist in jeder dem Stand der Technik entsprechenden Extraktionsapparatur realisierbar. Dabei erlaubt die Extraktion in z.B. einer Gegenstromkolonne die Minimierung der Extraktionsmittelmenge.

Im einzelnen ist das Verfahren der Erfindung nunmehr dadurch gekennzeichnet, daß man aus der verunreinigten Katalysatorlösung den Carbonylkomplex des Rhodiums sowie Essigsäure, Essigsäureanhydrid und Ethylidendiacetat mit einem Trialkylphosphan der Formel R¹R²R³P, wobei R¹, R², R³ gleich oder ungleich sind und C₃- bis C₈-Alkyl bedeuten, extrahiert und die Trialkylphosphan-Phase von der von Rhodium befreiten, die organischen Verunreinigungen enthaltenden Promotor-Phase abtrennt; die Trialkylphosphan-Phase in die flüchtigen Anteile Essigsäure, Essigsäureanhydrid und Ethylidendiacetat sowie Trialkylphosphan und den als Rückstand verbleibenden Carbonylkomplex des Rhodiums auftrennt; das zurückgewonnene Trialkylphosphan zur erneuten Extraktion einsetzt; daß man die verunreinigte, vom Carbonylkomplex des Rhodiums befreite Promotor-Phase durch Extraktion mit Dialkylethern, Carbonsäureestern oder Kohlenwasserstoffen von den organischen Verunreinigungen befreit und nach Abtrennen der Phase des Extraktionsmittels mit dem zurückgewonnenen Gemisch aus Essigsäure, Essigsäureanhydrid und Ethylidendiacetat sowie dem Carbonylkomplex des Rhodiums wieder vereinigt und dem Carbonylierungsreaktor zuführt; und daß man die als Rückstand einer Destillation der abgetrennten Phase des Extraktionsmittels verbleibenden organischen Verunreinigungen ausschleust.

Das Verfahren der Erfindung kann weiterhin bevorzugt und wahlweise dadurch gekennzeichnet sein, daß man
a) die Extraktion der verunreinigten Katalysatorlösung mit Trialkylphosphan bei Temperaturen von 20 - 100°C durchführt;
b) je Gewichtsteil verunreinigter Katalysatorlösung 0,5 - 10 Gewichtsteile Trialkylphosphan einsetzt;
c) je Gewichtsteil verunreinigter Katalysatorlösung 0,03 - 0,4 Gewichtsteile Methanol zusetzt;
d) aus der verunreinigten Katalysatorlösung zuerst die flüchtigen Anteile Essigsäure, Essigsäureanhydrid und Ethylidendiacetat abdestilliert, anschließend den Destillationsrückstand mit Trialkylphosphan unter Zusatz von Methanol extrahiert und das gebildete Zweiphasengemisch in gereinigte, den Carbonylkomplex des Rhodiums enthaltende Trialkylphosphan-Phase und organische Verunreinigungen enthaltende Promotor-Phase auftrennt und weiter wie oben beschrieben verfährt.
e) als Trialkylphosphan Tri-n-butylphosphan, Tri-n-octylphosphan oder 2-Butyl-di-n-octyl-phosphan einsetzt.

Die aus einem Carbonylierungsreaktor abfließende Reaktionsmischung wird destillativ in die gewünschten Endprodukte, insbesondere Essigsäureanhydrid und Essigsäure, sowie nichtumgesetzte, im Kreislauf geführte Ausgangsstoffe einerseits und die als Sumpfprodukt anfallende und im Kreislauf geführte Katalysatorlösung andererseits aufgetrennt. Ein Teilstrom dieser mit der Zeit durch undestillierbare organische Produkte verunreinigten Katalysatorlösung, welche je nach Verfahrensbedingungen bis zu 75 M-% (= Masse-%) an Essigsäureanhydrid, Essigsäure und/oder Ethylidendiacetat enthalten kann, wird aus dem Kreislauf der Katalysatorlösung entnommen und der Reinigung zugeführt. Die Katalysatorlösung enthält das Edelmetall Rhodium als Carbonylkomplex wie z.B.

[CH₃P(C₄H₉)₃] Rh(CO)I₄ oder [CH₃P(C₄H₉)₃] Rh(CO)₂I₂.

Die Katalysatorlösung kann ferner als organische Promotoren bevorzugt Tri-n-butyl-methyl-phosphoniumiodid oder Tetra-n-butylphosphoniumiodid enthalten.

Die ausgeschleuste, verunreinigte Katalysatorlösung wird mit Trialkylphosphan bevorzugt bei 20 bis 100°C extrahiert. Hierbei werden der Rhodium-Carbonylkomplex sowie die in der Katalysatorlösung befindlichen Anteile Essigsäure (AcOH), Essigsäureanhydrid (Ac₂O) und Ethylidendiacetat (EDA) extrahiert, während die undestillierbaren organischen Verunreinigungen mit dem Promotor als Promotor-Phase zurückbleiben. Aus der Trialkylphosphan(TAP)-Phase werden das AcOH/Ac₂O/EDA-Gemisch und das TAP durch Destillation zurückgewonnen, während der Rhodium-Carbonylkomplex als Rückstand anfällt. Das TAP wird zur erneuten Extraktion eingesetzt. Aus der Promotor-Phase werden die organischen Verunreinigungen mittels Extraktion mit z.B. (Methylacetat oder Di-i-propylether entfernt. Die vom Rückstand befreite Promotor-Phase wird sodann mit dem redestillierten AcOH-Ac₂O-EDA-Gemisch und dem als Destillationsrückstand verbliebenen gereinigten Rhodium-Carbonylkomplex vereinigt und der Carbonylierung als gereinigte Katalysatorlösung wieder zugeführt. Nach Abtrennen des Extraktionsmittels Methylacetat oder Di-i-propylether werden die organischen Verunreinigungen z .B. in einer Verbrennungsanlage vernichtet. Die zurückgewonnenen Extraktionsmittel werden wiederverwendet. Das Verfahren der Erfindung kann in sowohl kontinuierlicher als auch diskontinuierlicher Betriebsweise durchgeführt werden.

### Beispiel 1

Zur Entfernung der organischen Verunreinigungen werden dem Katalysatorkreislauf der Methylacetatcarbonylierung 500 g Katalysatorlösung der Zusammensetzung 6,1 M-% Rhodiumcarbonylkomplex [CH₃P(C₄H₉)₃] [Rh(CO)₂I₂] (≙ 5,0 g Rh = 1,0 M-%), 65,5 M-% Methyl-tri-n-butylphosphoniumiodid (TBPMeI) 3,0 M-% organische Verunreinigungen und 25,4 M-% eines Gemisches aus Essigsäure, Essigsäureanhydrid und Ethylidendiacetat entnommen und mit 1.200 g Tri-n-butylphosphan (TBP) bei 35°C extrahiert. Die TBP-Phase wird von der Promotor-Phase abgetrennt und destillativ in ein Essigsäure-Essigsäureanhydrid-Ethylidendiacetat-Gemisch, Tri-n-butylphosphan und Rhodium-Carbonylkomplex aufgetrennt. Das redestillierte Tri-n-butylphosphan wird zur erneuten Extraktion eingesetzt. Aus der TBPMeI-Phase (Promotor-Phase) werden durch Extraktion mit 750 ml Methylacetat, Abtrennen und anschließende Destillation der Methylacetat-Phase 13,5 g organische Verunreinigungen als Rückstand (Rh-Gehalt < 0,01 %) entfernt. Das dabei zurückgewonnene Methylacetat wird zur erneuten Extraktion verwendet. Das aus der TBPPhase erhaltene Destillat aus 127 g Essigsäure-Essigsäureanhydrid-Ethylidendiacetat-Gemisch wird mit dem von den organischen Verunreinigungen befreiten Rhodiumcarbonylkomplex und der gereinigten TBPMeI-Phase vereinigt und dem Katalysator-Kreislauf als 487 g gereinigte Lösung mit einem Rhodiumgehalt von 5 g wieder zugeführt. Die Rückführungsrate des Rhodiums in den Carbonylierungsprozeß beträgt nach der Reinigung der abgezogenen verunreinigten Katalysatorlösung > 99,97 %.

### Beispiel 2

Zur Entfernung der organischen Verunreinigungen werden dem Katalysator-Kreislauf der Methanol/Methylacetatcarbonylierung 500 g Katalysatorlösung der Zusammensetzung 6,9 M-% Rhodium-Carbonylkomplex [CH₃P(C₄H₉)₃] [Rh(CO)I₄] (≙ 4,0 g Rh = 0,8 M.-%), 55,3 M.-% Methyl-tri-n-butylphosphoniumiodid (TBPMeI), 2,4 M.-% organische Verunreinigungen und 35,4 % eines Gemisches aus Essigsäure, Essigsäureanhydrid und Ethylidendiacetat entnommen und mit 1.000 g Tri-n-butylphosphan (TBP) unter Zusatz von 60 g Methanol bei 25°C extrahiert. Die TBP-Phase wird von der TBPMeI-Phase (Promotor-Phase) abgetrennt und destillativ in ein Essigsäure-Essigsäureanhydrid-Ethylidendiacetat-Gemisch, Tri-n-butylphosphan und Rhodiumcarbonylkomplex aufgetrennt. Das redestillierte TBP wird zur erneuten Extraktion eingesetzt. Aus der TBPMeI-Phase werden durch Extraktion mit 1.000 ml Di-i-propylether, Abtrennen und anschließende Destillation der Di-i-propylether-Phase 10,7 g organische Verunreinigungen als Rückstand (Rh-Gehalt < 0,01 %) entfernt. Das dabei zurückgewonnene Methanol und der Di-i-propylether werden in die jeweilige Extraktionsstufe rückgeführt. Das aus der TBP-Phase erhaltene Destillat aus 177 g Essigsäure-Essigsäureanhydrid-Ethylidendiacetat-Gemisch wird mit dem von den organischen Verunreinigungen befreiten Rhodiumcarbonylkomplex und der gereinigten TBPMeI-Phase vereinigt und dem Katalysator-Kreislauf als 489 g gereinigte Lösung mit einem Rhodiumgehalt von 4 g wieder zugeführt. Die Rückführungsrate des Rhodiums in den Carbonylierungsprozeß beträgt nach der Reinigung der abgezogenen verunreinigten Katalysatorlösung > 99,97 %.

### Beispiel 3

Zur Entfernung der organischen Verunreinigungen werden dem Katalysatorkreislauf der Methanol/Methylacetatcarbonylierung 500 g Katalysatorlösung der Zusammensetzung 6,1 M.-% Rhodiumcarbonylkomplex [CH₃P(C₄H₉)₃] [Rh(CO)₂I₂] (entsprechend 5 g Rhodium = 1,0 M.-%), 65,0 M.-% Methyl-tri-n-butylphosphoniumiodid (TBPMeI), 3,0 M.-% organische Verunreinigungen und 25,9 M.-% eines Gemisches aus Essigsäure und Essigsäureanhydrid entnommen und mit 1.000 g Tri-n-octylphosphan (TOP) bei 60°C extrahiert. Die TOP-Phase wird von der Promotorphase abgetrennt und destillativ in ein Essigsäure/Essigsäureanhydrid-Gemisch, Tri-n-octylphosphan und Rhodiumcarbonylkomplex aufgetrennt. Das redestillierte Tri-n-octylphosphan wird zur erneuten Extraktion eingesetzt. Aus der TBPMeI-Phase (Promotorphase) werden durch Extraktion mit 800 ml Methylacetat, Abtrennen und anschließende Destillation der Methylacetat-Phase 13,5 g organische Verunreinigungen als Rückstand (Rh-Gehalt < 0,01%) entfernt. Das dabei zurückgewonnene Methylacetat wird zur erneuten Extraktion verwendet. Das aus der TOP-Phase erhaltene Destillat aus 129 g Essigsäure/Essigsäureanhydrid-Gemisch wird mit dem von den organischen Verunreinigungen befreiten Rhodiumcarbonylkomplex und der gereinigten TBPMeI-Phase vereinigt und dem Katalysatorkreislauf als 487 g gereinigte Lösung mit einem Rhodiumgehalt von 5 g wieder zugeführt. Die Rückführungsrate des Rhodiums in den Carbonylierungsprozeß beträgt nach der Reinigung der abgezogenen verunreinigten Katalysatorlösung > 99,97 %.

### Beispiel 4

Zur Entfernung der organischen Verunreinigungen werden dem Katalysatorkreislauf der Methanol/Methylacetatcarbonylierung 500 g Katalysatorlösung der Zusammensetzung 6,9 M.-% Rhodiumcarbonylkomplex [CH₃P(C₄H₉)₃] [Rh(CO)I₄] (entsprechend 4 g Rhodium = 0,8 M.-%), 59,0 M.-% Methyl-tri-n- butylphosphoniumiodid (TBPMeI) , 2,4 M.-% organische Verunreinigungen und 31,7 M.-% eines Gemisches aus Essigsäure und Essigsäureanhydrid entnommen und mit 11oo g 2-Butyl-di-n-octylphosphan (BDOP) bei 5o°C extrahiert. Die BDOP-Phase wird von der TBPMeI-Phase (Promotorphase) abgetrennt und destillativ in ein Essigsäure/Essigsäureanhydrid-Gemisch, 2-Butyl-di-n-octylphosphan und Rhodiumcarbonylkomplex aufgetrennt. Das redestillierte BDOP wird zur erneuten Extraktion eingesetzt. Aus der TBPMeI-Phase werden durch Extraktion mit 9oo ml Diisopropylether, Abtrennen und anschließende Destillation der Diisopropylether-Phase 1o,7 g organische Verunreinigungen alsRückstand (Rh-Gehalt < 0,01 %) entfernt. Der zurückgewonnene Diisopropylether wird in die Extraktionsstufe rückgeführt. Das aus der BDOP-Phase erhaltene Destillat aus 158 g Essigsäure/Essigsäureanhydrid-Gemisch wird mit dem von den organischen Verunreinigungen befreiten Rhodiumcarbonylkomplex und der gereinigten TBPMeI-Phase vereinigt und dem Katalysatorkreislauf als 489 g gereinigte Lösung mit einem Rhodiumgehalt von 4 g wieder zugeführt. Die Rückführungsrate des Rhodiums in den Carbonylierungsprozeß beträgt nach der Reinigung der abgezogenen verunreinigten Katalysatorlösung > 99,97 %.

## Patentansprüche

1. Verfahren zur Reinigung und Rückgewinnung der bei der Carbonylierung von Methanol und/oder Methylacetat und/oder Dimethylether anfallenden verunreinigten Katalysatorlösung, welche Carbonylkomplexe des Rhodiums, quaternäre Organophosphorverbindungen als organische Promotoren, undestillierbare organische Verunreinigungen sowie Essigsäure, Essigsäureanhydrid und Ethylidendiacetat enthält, dadurch gekennzeichnet, daß man aus der verunreinigten Katalysatorlösung den Carbonylkomplex des Rhodiums sowie Essigsäure, Essigsäureanhydrid und Ethylidendiacetat mit einem Trialkylphosphan der Formel R¹R²R³P, wobei R¹, R², R³ gleich oder ungleich sind und C₃- bis C₈-Alkyl bedeuten, extrahiert und die Trialkylphosphan-Phase von der von Rhodium befreiten, die organischen Verunreinigungen enthaltenden Promotor-Phase abtrennt; die Trialkylphosphan-Phase in die flüchtigen Anteile Essigsäure, Essigsäureanhydrid und Ethylidendiacetat sowie Trialkylphosphan und den als Rückstand verbleibenden Carbonylkomplex des Rhodiums auftrennt; das zurückgewonnene Trialkylphosphan zur erneuten Extraktion einsetzt; daß man die verunreinigte, vom Carbonylkomplex des Rhodiums befreite Promotor-Phase durch Extraktion mit Dialkylethern, Carbonsäureestern oder Kohlenwasserstoffen von den organischen Verunreinigungen befreit und nach Abtrennen der Phase des Extraktionsmittels mit dem zurückgewonnenen Gemisch aus Essigsäure, Essigsäureanhydrid und Ethylidendiacetat sowie dem Carbonylkomplex des Rhodiums wieder vereinigt und dem Carbonylierungsreaktor zuführt; und daß man die als Rückstand einer Destillation der abgetrennten Phase des Extraktionsmittels verbleibenden organischen Verunreinigungen ausschleust.

2. Verfahren nach Anspruch 1, dadurch gekennzeichnet, daß man die Extraktion der verunreinigten Katalysatorlösung mit Trialkylphosphan bei Temperaturen von 20 bis 100°C durchführt.

3. Verfahren nach mindestens einem der Ansprüche 1 und 2, dadurch gekennzeichnet, daß man je Gewichtsteil verunreinigter Katalysatorlösung 0,5 bis 10 Gewichtsteile Trialkylphosphan einsetzt.

4. Verfahren nach mindestens einem der vorhergehenden Ansprüche, dadurch gekennzeichnet, daß man je Gewichtsteil verunreinigter Katalysatorlösung 0,03 bis 0,4 Gewichtsteile Methanol zusetzt.

5. Verfahren nach mindestens einem der vorhergehenden Ansprüche, dadurch gekennzeichnet, daß man aus der verunreinigten Katalysatorlösung zuerst die flüchtigen Anteile Essigsäure, Essigsäureanhydrid und Ethylidendiacetat abdestilliert, anschließend den Destillationsrückstand mit Trialkylphosphan unter Zusatz von Methanol extrahiert und das gebildete Zweiphasengemisch in gereinigte, den Carbonylkomplex des Rhodiums enthaltende Trialkylphosphan-Phase und organische Verunreinigungen enthaltende Promotor-Phase auftrennt und weiter nach Anspruch 1 verfährt.

6. Verfahren nach mindestens einem der vorhergehenden Ansprüche, dadurch gekennzeichnet, daß man als Trialkylphosphan Tri-n-butylphosphan, Tri-n-octylphosphan oder 2-Butyl-di-n-octylphosphan einsetzt.

## Claims

1. A process for purifying and recovering the contaminated catalyst solution arising in the carbonylation of methanol and/or methyl acetate and/or dimethyl ether and containing carbonyl complexes of rhodium, quaternary organophosphorus compounds as organic promoters, undistillable organic impurities as well as acetic acid, acetic anhydride and ethylidene diacetate, which comprises extracting the carbonyl complex of rhodium as well as acetic acid, acetic anhydride and ethylidene diacetate from the contaminated catalyst solution by means of a trialkylphosphane of the formula R¹R²R³P, in which R¹, R² and R³ are identical or different and are C₃- to C₈-alkyl, and separating the trialkylphosphane phase from the promoter phase freed of rhodium and containing the organic impurities; separating the trialkylphosphane phase into the volatile constituents acetic acid, acetic anhydride and ethylidene diacetate as well as trialkylphosphane and the carbonyl complex of rhodium remaining as the residue; using the recovered trialkylphosphane for further extraction, freeing the contaminated promoter phase, freed of the carbonyl complex of rhodium, of the organic impurities by extraction with dialkyl ethers, carboxylic acid esters or hydrocarbons and, after the extractant phase has been separated off, recombining it with the recovered mixture of acetic acid, acetic anhydr-ide and ethylidene diacetate and with the carbonyl complex of rhodium and feeding it to the carbonylation reactor; and removing the organic impurities, remaining as the residue of a distillation of the extractant phase, which has been separated off, from the system.

2. The process as claimed in claim 1, wherein the extraction of the contaminated catalyst solution is carried out with a trialkylphosphane at temperatures from 20 to 100°C.

3. The process as claimed in at least one of claims 1 and 2, wherein 0.5 to 10 parts by weight of trialkylphosphane are used per part by weight of contaminated catalyst solution.

4. The process as claimed in at least one of the preceding claims, wherein 0.03 to 0.4 part by weight of methanol is added per part by weight of contaminated catalyst solution.

5. The process as claimed in at least one of the preceding claims, wherein the volatile constituents acetic acid, acetic anhydride and ethylidene diacetate are first distilled off from the contaminated catalyst solution, the distillation residue is then extracted with trialkylphosphane with an addition of methanol and the two-phase mixture formed is separated into a purified trialkylphosphane phase containing the carbonyl complex of rhodium and a promoter phase containing organic impurities, and the further procedure is as claimed in claim 1.

6. The process as claimed in at least one of the preceding claims, wherein tri-n-butylphosphane, tri-n-octylphosphane or 2-butyl-di-n-octylphosphane is used as the trialkylphosphane.

## Revendications

1. Procédé pour purifier et récupérer la solution de catalyseur impure obtenue à la carbonylation du méthanol et/ou de l'acétate de méthyle et/ou de l'éther diméthylique, solution qui contient des complexes carbonylés du rhodium, des composés organophosphorés quaternaires en tant qu'activateurs organiques, des impuretés organiques non distillables ainsi que de l'acide acétique, de l'anhydride acétique et de l'éthylidène-diacétate, caractérisé en ce que l'on extrait de la solution de catalyseur impure le complexe carbonylé du rhodium ainsi que l'acide acétique, l'anhydride acétique et l'éthylidène-acétique à l'aide d'un trialkylphosphane de formule R¹R²R³P dans laquelle R¹, R², R³, ayant les significations identiques ou différentes, représentent chacun un groupe alkyle en C₃-C₈, et on sépare la phase trialkylphosphane de la phase activateur débarrassée du rhodium mais contenant les impuretés organiques ; on sépare la phase trialkylphosphane en les fractions volatiles acide .acétique, anhydride acétique et éthylidène-diacétate, le trialkylphosphane et, en résidu, le complexe carbonylé du rhodium ; on renvoie le trialkylphosphane récupéré à l'extraction ; on débarrasse des impuretés organiques la phase activateur impure, débarrassée du complexe carbonylé du rhodium, par extraction à l'aide d'éthers dialkyliques, d'esters d'acides carboxyliques ou d'hydrocarbures et, après séparation de la phase de l'agent d'extraction, on la combine à nouveau avec le mélange récupéré d'acide acétique, d'anhydride acétique et d'éthylidène-diacétate ainsi qu'avec le complexe carbonylé du rhodium et on renvoie au réacteur de carbonylation ; et on évacue les impuretés organiques restant en résidu d'une distillation de la phase de l'agent d'extraction séparée.

2. Procédé selon la revendication 1, caractérisé en ce que l'on procède à l'extraction de la solution de catalyseur impure par le trialkylphosphane à des températures de 20 à 100°C.

3. Procédé selon au moins une des revendications 1 et 2, caractérisé en ce que l'on utilise de 0,5 à 10 parties en poids de trialkylphosphane par partie en poids de la solution de catalyseur impure.

4. Procédé selon au moins une des revendications qui précèdent, caractérisé en ce que l'on ajoute de 0,03 à 0,4 partie en poids de méthanol par partie en poids de la solution de catalyseur impure.

5. Procédé selon au moins une des revendications qui précèdent, caractérisé en ce que, à partir de la solution de catalyseur impure, on distille d'abord les fractions volatiles : acide acétique, anhydride acétique et éthylidène-diacétate, on extrait ensuite le résidu de distillation par le trialkylphosphane avec addition de méthanol et on sépare le mélange à deux phases ainsi formé en une phase trialkylphosphane purifiée, contenant le complexe carbonylé du rhodium, et une phase activateur contenant les impuretés organiques, et on poursuit comme décrit dans la revendication 1.

6. Procédé selon au moins une des revendications qui précèdent, caractérisé en ce que l'on utilise en tant que trialkylphosphane le tri-n-butylphosphane, le tri-n-octylphosphane ou le 2-butyl-di-n-octylphosphane.
